(19) Europäisches Patentamt
European Patent Office
Office européen des brevets

(11) **EP 3 179 249 A1**

(12) **EUROPEAN PATENT APPLICATION**
published in accordance with Art. 153(4) EPC

(43) Date of publication:
**14.06.2017 Bulletin 2017/24**

(51) Int Cl.:
*G01N 33/49* (2006.01)     *C12Q 1/02* (2006.01)
*G01N 21/27* (2006.01)     *G01N 33/48* (2006.01)
*G01N 33/483* (2006.01)    *C12Q 1/68* (2006.01)

(21) Application number: **15830123.4**

(22) Date of filing: **19.06.2015**

(86) International application number:
**PCT/JP2015/067712**

(87) International publication number:
**WO 2016/021311 (11.02.2016 Gazette 2016/06)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA**

(30) Priority: **05.08.2014 JP 2014159346**

(71) Applicant: **Fujifilm Corporation**
**Minato-ku**
**Tokyo 106-8620 (JP)**

(72) Inventors:
• **ISHII, Yasuyuki**
  **Kanagawa 258-8538 (JP)**
• **MATSUBARA, Kenta**
  **Kanagawa 258-8538 (JP)**
• **FUKUI, Shinichiro**
  **Kanagawa 258-8538 (JP)**

(74) Representative: **Klunker . Schmitt-Nilson . Hirsch**
**Patentanwälte**
**Destouchesstraße 68**
**80796 München (DE)**

(54) **NUCLEATED ERYTHROCYTE SORTING METHOD**

(57)     Provided is a method for sorting nucleated red blood cells which is capable of reliably separating fetus-derived nucleated red blood cells from maternal blood. The method includes: a candidate cell selection step of selecting a candidate cell of nucleated red blood cells in accordance with shapes of cells; a white blood cell selection step of setting a search range including the candidate cell and selecting a white blood cell within the search range; a nucleated red blood cell definition step of defining that the candidate cell is a nucleated red blood cell through comparison of spectral characteristics of hemoglobin between the candidate cell and the white blood cell; and a sorting step of sorting the nucleated red blood cells into mother-derived nucleated red blood cells or fetus-derived nucleated red blood cells through comparison of an allele contained in the nucleated red blood cell after the nucleated red blood cell definition step with an allele contained in a white blood cell which has not been selected in the candidate cell selection step or in the white blood cell selected in the white blood cell selection step.

FIG. 2

```
┌──────────────────────────┐
│   CANDIDATE CELL         │
│   SELECTION STEP         │─── S32
└──────────────────────────┘
            │
            ▼
┌──────────────────────────┐
│   WHITE BLOOD CELL       │
│   SELECTION STEP         │─── S34
└──────────────────────────┘
            │
            ▼
┌──────────────────────────┐
│  NUCLEATED RED BLOOD CELL│
│   DEFINITION STEP        │─── S36
└──────────────────────────┘
```

**Description**

**BACKGROUND OF THE INVENTION**

1. Field of the Invention

The present invention relates to a method for sorting nucleated red blood cells, and particularly to a method for sorting nucleated red blood cells in which fetus-derived nucleated red blood cells in blood of a pregnant mother is specified using information of the shape of a cell, optical information, and genetic information.

2. Description of the Related Art

In the related art, an amniotic fluid test of checking a chromosome of a fetal cell in amniotic fluid has been performed through amniocentesis as a prenatal diagnosis. However, in this method, a possibility of miscarriage is pointed out as a problem.

In contrast, it has been recently found that a fetal cell is transferred into blood of a pregnant mother (hereinafter, also simply referred to as a "mother") and circulates with blood in the mother. Therefore, it is possible to realize a prenatal diagnosis which has a low possibility of miscarriage and does not require amniocentesis if it is possible to reliably analyze deoxyribonucleic acid (DNA) of a chromosome of a fetal cell in blood of the mother with excellent reproducibility, using maternal blood.

However, approximately only one fetal cell (nucleated red blood cell) existing in maternal blood exists in the number (mL) of maternal blood (1 mL = $10^{-6}$ $m^3$). Reliably obtaining such an extremely a small number of fetal cells (nucleated red blood cells) is a significantly large problem in a case of performing a prenatal diagnosis using maternal blood.

Regarding such a problem, a method for concentrating nucleated red blood cells, for example, a technique of removing plasma components and red blood cell components of a mother through density gradient centrifugation is disclosed in WO2012-023298A described below. In addition, nucleated red blood cells as fetal cells in maternal blood are obtained through a technique (magnetic activated cell sorting: MACS method) of magnetically separating white blood cells of a mother from blood using an antibody which immunologically reacts specifically to proteins on surfaces of the white blood cells, a technique (fluorescence activated cell sorting: FACS method) of separating nucleated red blood cells of a fetus from blood using a fluorescent coloring agent and an antibody which immunologically reacts specifically to a γ chain of fetal hemoglobin, and the like are disclosed in Y. L. Zheng et al. "Prenatal diagnosis from maternal blood: simultaneous immunophenotyping and FISH of fetal nucleated erythrocytes isolated by negative magnetic cell sorting" J Med. Genet. 1993 Dec; 30 (12): 1051-1056 and Y. L. Zheng et al. "Flow sorting of fetal erythroblasts using intracytoplasmic anti-fetal haemoglobin: preliminary observations on maternal samples" Prenat Diagn 1995 Oct; 15 (10): 897-905.

These methods are useful techniques for increasing the probability of obtaining target cells by increasing the concentration of fetus-derived nucleated red blood cells in blood. However, since there are still mother-derived blood cells around fetus-derived nucleated red blood cells, it has not been possible to reliably obtain fetus-derived nucleated red blood cells within a short period of time.

In addition, a technique of obtaining required cells after identifying the types of cells using optical information is disclosed. For example, a method for separating fetus-derived nucleated red blood cells from maternal cells containing mother-derived nucleated red blood cells through a method for reacting a mouse fetal hemoglobin antibody with blood containing fetus-derived nucleated red blood cells with which a slide glass is coated, subsequently reacting the reactant with a biotin-conjugated goat anti-mouse antibody, adding alkaline phosphatase (ALP)-conjugated streptavidin thereto, and then, adding a vector BLUE substrate thereto is disclosed in JP2002-514304A. A device of identifying the types of blood cells using light at a wavelength of 415 nm in the vicinity of the maximum absorption wavelength of hemoglobin is disclosed in JP1983-115346A (JP-S58-115346A). Biological information analysis in which optical characteristics of biological tissue are used and a device of detecting an oxygen administration state of tissue using the difference in the absorbance of light between oxygenated hemoglobin and reduced hemoglobin are disclosed in JP2014-14485A. A nucleated cell search automation device intended to coat slide glass with blood, identify a fetus cell in accordance with the form thereof, and individually collect only fetal cells is disclosed in JP2004-248619A. A biochemical inspection device for analyzing biochemical substances, microorganisms, cells, or the like within a sample is disclosed in JP2004-361158A.

SUMMARY OF THE INVENTION

The inventions disclosed in JP2002-514304A, JP1983-115346A (JP-S58-115346A), JP2014-14485A, and JP2004-248619A are useful as techniques which can separate nucleated red blood cells. However, in JP2002-514304A, since hemoglobin exists in cytoplasm, hemoglobin is separated by a cell membrane from the outside, and therefore, it was not found whether or not an immunological reaction is caused in fetus-derived nucleated red blood cells with high efficiency. In JP1983-115346A (JP-S58-115346A), red blood cells and white blood cells are separated from each other in a solution. However, it is impossible to sufficiently perform the separation, and therefore, mixing of red blood cells with white blood cells is caused. JP2014-14485A and JP2004-248619A are techniques of separating cells using the shapes of cells and optical information. However, there is no disclosure of separation of different solid-derived nucleated red blood cells of mother-derived cells and fetus-derived cells. Accordingly, there is still a problem to reliably separate fetus-derived nucleated red blood cells from mother-derived nucleated red blood cells. In addition, there is also a case where a white blood cell is selected as a candidate cell of a nucleated red blood cell in addition to separating nucleated red blood cells into fetus-derived nucleated red blood cells. Therefore, it is necessary to separate red blood cells and white blood cells from the candidate cell.

The present invention has been made in consideration of such circumstances, and an object of the present invention is to provide a method for sorting nucleated red blood cells in which it is possible to reliably separate fetus-derived nucleated red blood cells from maternal blood.

In order to achieve the purpose of the present invention, according to an aspect of the present invention, there is provided a method for sorting nucleated red blood cells, the method comprising: a candidate cell selection step of selecting a candidate cell of nucleated red blood cells in accordance with shapes of cells; a white blood cell selection step of setting a search range including the candidate cell and selecting a white blood cell within the search range; a nucleated red blood cell definition step of defining that the candidate cell is a nucleated red blood cell through comparison of spectral characteristics of hemoglobin between the candidate cell and the white blood cell; and a sorting step of sorting the nucleated red blood cells into mother-derived nucleated red blood cells or fetus-derived nucleated red blood cells through comparison of an allele contained in the nucleated red blood cell after the nucleated red blood cell definition step with an allele contained in a white blood cell which has not been selected in the candidate cell selection step or in the white blood cell selected in the white blood cell selection step.

According to the method for sorting nucleated red blood cells of the present invention, it is possible to determine whether or not a candidate cell is a nucleated red blood cell by selecting the candidate cell of the nucleated red blood cell, setting a range including this candidate cell of the nucleated red blood cell as a search range, searching a white blood cell within this search range, and comparing the candidate cell with the white blood cell.

In the related art, there is also a case where all candidate cells are white blood cell-derived cells when it is impossible to prevent mixing in of the white blood cell as a candidate cell. In this case, if it is confirmed that there is no nucleated red blood cell, in particular, a fetus-derived nucleated red blood cell after checking genetic information, it is necessary to start over from the sorting out of a candidate cell. Therefore, it is inefficient and it takes time.

According to the above-described method for sorting nucleated red blood cells, it is possible to more reliably promptly define a fetus-derived nucleated red blood cell in the next step at lower costs since the candidate cell is defined as a nucleated red blood cell through the nucleated red blood cell definition step and a white blood cell-derived cell is not mixed with the candidate cell.

In addition, in the sorting step, an allele of the nucleated red blood cell after the nucleated red blood cell definition step is compared with an allele of the white blood cell which has been known in the step previous to the sorting step. Since the white blood cell is a mother-derived blood component, it is possible to sort out a nucleated red blood cell having an allele different from that of the white blood cell as a fetus-derived nucleated red blood cell and to sort out a nucleated red blood cell having an allele almost the same as that of the white blood cell as a mother-derived nucleated red blood cell.

In order to achieve the purpose of the present invention, according to another aspect of the present invention, there is provided a method for sorting nucleated red blood cells, the method comprising: a candidate cell selection step of selecting a candidate cell of nucleated red blood cells in accordance with shapes of cells; a white blood cell selection step of setting a search range including the candidate cell and selecting a white blood cell within the search range; a nucleated red blood cell definition step of defining that the candidate cell is a nucleated red blood cell through comparison of spectral characteristics of hemoglobin between the candidate cell and the white blood cell; and a sorting step of sorting the nucleated red blood cells into mother-derived nucleated red blood cells or fetus-derived nucleated red blood cells by checking presence or absence of a Y chromosome contained in the nucleated red blood cells after the nucleated red blood cell definition step.

According to the method for sorting nucleated red blood cells of the present invention, it is possible to determine whether or not a candidate cell is a nucleated red blood cell by selecting the candidate cell of the nucleated red blood cell, setting a range including this candidate cell of the nucleated red blood cell as a search range, searching a white blood cell within this search range, and comparing the candidate cell with the white blood cell.

In addition, in a case where a fetal cell is a male infant, it is possible to determine that a nucleated red blood cell is a fetus-derived nucleated red blood cell by checking the presence of a Y chromosome in the nucleated red blood cell after the nucleated red blood cell definition step, in the sorting step.

In still another aspect of the present invention, it is preferable that the search range is a range which is set stepwise from the candidate cell based on a size of the candidate cell, the white blood cell within the search range is selected in the white blood cell selection step, and the search range is widened in accordance with the number of the selected white blood cells.

This aspect prescribes a method for setting the search range which can be set stepwise based on the size of candidate cell. In a case where it is impossible to select the desired number of white blood cells within a search range which has been set first after setting the search range stepwise, it is possible to set the number of white blood cells to a desired value by widening the search range stepwise. It is possible to average optical information of the white blood cells by increasing the number of white blood cells. Therefore, it is possible to average a standard which is different from those of the nucleated red blood cells to be sorted out and to suppress variation.

In still another aspect of the present invention, it is preferable that the search range is a range in which a white blood cell having a distance from the candidate cell is shortest and a white blood cell having a distance from the candidate cell becomes long are sequentially selected, and a number of the white blood cells which have been selected is 2 to 20.

This aspect prescribes another method for setting the search range in which white blood cells can be sequentially selected starting from a white blood cell of which the distance from a candidate cell is shortest. The number of white blood cells is 2 to 20. This range is preferable since it is possible to average optical information of the white blood cells and to average optical information as the number of white blood cells is larger. It is preferable that the number of white blood cells is 3 to 10 since it takes time for measurement of the white blood cells as the number of white blood cells is larger.

The "distance of a white blood cell from a candidate cell" is a distance between the center of the candidate cell and the center of the white blood cell or a distance between the center of gravity of the candidate cell and the center of gravity of the white blood cell.

In still another aspect of the present invention, it is preferable that the spectral characteristics are absorbances in at least one monochromatic light beam selected from wavelengths included in a light wavelength region of 400 to 650 nm.

Hemoglobin shows absorption of light in a light wavelength region of 400 to 650 nm, and therefore, it is possible to clarify the difference with the absorbance of a white blood cell in a case where the candidate cell is a nucleated red blood cell, using the absorbance at a wavelength included in the light wavelength region of 400 to 650 nm. Accordingly, it is possible to determine whether or not the candidate cell is a nucleated red blood cell by comparing the candidate cell and the white blood cell.

In still another aspect of the present invention, it is preferable that the candidate cell selection step includes a first step of selecting a cell of which a ratio of an area of a cell nucleus of the cell to an area of cytoplasm of the cell satisfies Formula (1) (also referred to as Formula 1), in a case where the area of cytoplasm of the cell is set to C and the area of the cell nucleus of the cell is set to N.

$$0.25 < N / C < 1.0 \qquad (1)$$

In still another aspect of the present invention, it is preferable that the candidate cell selection step includes a second step of selecting a cell of which a ratio of an area of a cell nucleus to a square of a length of a major axis of a circular shape or a major axis of an elliptical shape satisfies Formula (2) (also referred to as Formula 2), in a case where the area of the cell nucleus of the cell is set to N and the length of the diameter of the circular shape circumscribing the cell nucleus of the cell or the length of the major axis of the elliptical shape is set to L.

$$0.65 < N / (L \times L) < 0.785 \qquad (2)$$

The above-described aspect prescribes specific conditions for selecting a candidate cell in accordance with the shape of a cell in the candidate cell selection step, and Formula (1) prescribes an area ratio of cytoplasm of a cell and a nucleus and Formula (2) prescribes a degree of circularity of a nucleus. Since there is a high possibility that a cell having a nucleus under the conditions of the above-described Formula (1) and the above-described Formula (2) may be a red blood cell, it is possible to increase a possibility that the candidate cell is a nucleated red blood cell.

According to the method for sorting nucleated red blood cells of the present invention, a nucleated red blood cell is sorted into a fetus-derived nucleated red blood cell or a mother-derived nucleated red blood cell in accordance with genetic information after selecting a cell which becomes a candidate of a nucleated red blood cell in accordance with the shape of the cell and defining the selected cell as a red blood cell in accordance with spectral characteristics. In a case of performing the sorting in accordance with genetic information, it is possible to obtain a nucleated red blood cell with which a white blood cell-derived cell is not mixed. Therefore, it is possible to more promptly reliably define a fetus-derived nucleated red blood cell at lower costs.

BRIEF DESCRIPTION OF THE DRAWINGS

Fig. 1 is a flow chart showing a procedure of a method for inspecting a chromosome of a fetus.
Fig. 2 is a flow chart showing a procedure of a definition step.
Fig. 3 is a flow chart showing a procedure of a candidate cell specification step.
Fig. 4A is a schematic view showing the shape of a cell to be selected.
Fig. 4B is a schematic view showing the shape of a cell to be removed.
Fig. 5 is a view illustrating a method for setting a search range based on the number of white blood cells.
Fig. 6 is a flow chart showing a procedure of setting the search range based on the number of white blood cells.
Fig. 7 is a view illustrating a method for setting a search range of white blood cells based on the area of a candidate cell.
Fig. 8 is a flow chart showing a procedure of setting the search range of white blood cells based on the area of the candidate cell.
Fig. 9 is a flow chart showing a procedure of a nucleated red blood cell definition step performed in accordance with optical characteristics.

DESCRIPTION OF THE PREFERRED EMBODIMENTS

Hereinafter, a method for sorting nucleated red blood cells according to the present invention will be described with reference to accompanying drawings. "To" in the present specification is used as a meaning including numerical values described before and after "to" as lower limit values and upper limit values.

[Method for Inspecting Chromosome of Fetus]

A method for sorting nucleated red blood cells according to the present invention will be described using a method for inspecting a chromosome of a fetus.

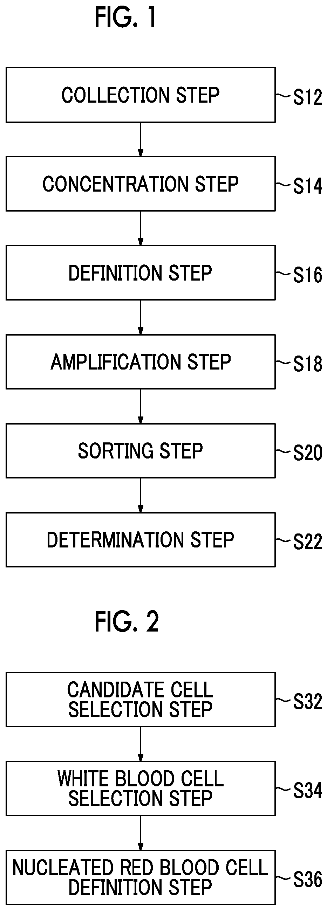

Fig. 1 is a flow chart showing a procedure of a method for inspecting a chromosome of a fetus. The method for inspecting a chromosome of a fetus includes a collection step (Step S12) of collecting maternal blood from a pregnant mother; a concentration step (Step S14) of concentrating nucleated red blood cells in the maternal blood; a sorting step (Step S16) of sorting the nucleated red blood cells in the maternal blood, in which the nucleated red blood cells are concentrated through the concentration step, in accordance with the shape of a cell, the shape of a nucleus, and spectral characteristics of hemoglobin; an amplification step (Step S18) of amplifying a nucleic acid of the chromosome of the nucleated red blood cells at least defined in the definition step; a sorting step (Step S20) of sorting the nucleated red blood cells into mother-derived nucleated red blood cells or fetus-derived nucleated red blood cells by comparing an allele of the amplified product which has been amplified through the amplification step with a mother-derived white blood cell or checking the presence or absence of a Y chromosome of the amplified product which has been amplified through the amplification step; and a determination step (Step S22) of determining the presence or absence of a numerical abnormality of the fetus-derived chromosome through comparison of the defined amount of the amplified product with a chromosome of a mother or a fetus which is known that there is no abnormality. The method for sorting nucleated red blood cells of the present invention includes the definition step (Step S16) and a sorting step (Step S20).

<collection Step (Step S12)>

The collection step is a step of collecting maternal blood which is a blood sample. It is preferable to use peripheral blood of a pregnant mother having no concern of invasion, as maternal blood.

Mother-derived nucleated red blood cells and fetus-derived nucleated red blood cells are contained in peripheral blood of a mother in addition to white blood cells, such as eosinophils, neutrophils, basophils, monocytes, and lymphocytes, which are derived from a mother, or mature red blood cells having no nucleus. It is known that the fetus-derived nucleated red blood cells exist in maternal blood after about 6 weeks of pregnancy. In the present embodiment in which a prenatal diagnosis is performed, peripheral blood of a mother after about 6 weeks of pregnancy is inspected.

Fetus-derived nucleated red blood cells are erythrocyte precursors which exist in blood of a mother after passing through the placenta. Red blood cells of a fetus can be nucleated during pregnancy of a mother. Since there are chromosomes in these red blood cells, it is possible to obtain fetus-derived chromosomes and fetus genes through less invasive means. It is known that these fetus-derived nucleated red blood cells exist at a ratio of one fetus-derived nucleated red blood cell to 106 cells in maternal blood. Therefore, the existence probability of the fetus-derived nucleated red blood cells in peripheral blood of a mother is significantly low.

<Concentration Step (Step S14)>

Next, concentration of nucleated red blood cells in maternal blood collected in the collection step is performed through a concentration step. The concentration step is preferably performed through density gradient centrifugation.

[Density Gradient Centrifugation]

The density gradient centrifugation is a method for performing separation using the difference in the density of components in blood. By selecting a first medium and a second medium so as to interpose density values of components to be separated, it is possible to collect target components (fetus-derived nucleated red blood cells in the present invention) at a boundary between the first medium and the second medium after the centrifugation. Moreover, it is possible to obtain blood in which red blood cells are concentrated, by collecting fractions existing at a boundary between the first medium and the second medium.

Specifically, the concentration of nucleated red blood cells can be performed through a method including a step of injecting the first medium into a bottom portion of a centrifuge tube, a step of cooling the centrifuge tube in which the first medium is stored, a step of stacking the second medium on the first medium which has been cooled within the centrifuge tube, a step of stacking peripheral blood of a mother, as a blood sample, on the second medium within the centrifuge tube, a step of subjecting the first medium, the second medium, and the blood sample which have been stored in the centrifuge tube, to centrifugation, and a step of collecting fractions containing fetus-derived nucleated red blood cells from a layer between the first medium and the second medium after the centrifugation.

The density of blood of a mother which contains nucleated red blood cells of a fetus is disclosed in WO2012/023298A. According to the disclosure, the density of fetus-derived nucleated red blood cells which is assumed is about 1.065 to 1.095 g/mL, the density of blood cells of a mother is about 1.070 to 1.120 g/mL in a case of red blood cells and about 1.090 to 1.110 g/mL in a case of eosinophils, about 1.075 to 1.100 g/mL in a case of neutrophils, about 1.070 to 1.080 g/mL in a case of basophils, about 1.060 to 1.080 g/mL in a case of lymphocytes, and about 1.060 to 1.070 g/mL in a case of monocytes. It is easy to convert the numerical values of the density from a unit represented by grams per milliliter (g/mL) to an SI unit system, and 1 gram per milliliter (g/mL) is 1000 kilograms per cubic meter ($kg/m^3$).

The density of media (first medium and second medium) is set so as to separate fetus-derived nucleated red blood cells having a density of 1.065 to 1.095 g/mL from other blood cell components in a mother. The density of the center of the fetus-derived nucleated red blood cells is about 1.080 g/mL. Therefore, it is possible to collect desired fetus-derived nucleated red blood cells at a boundary of the density by producing two media which have different densities interposing the density and making the media adjacently overlap each other. It is preferable to set the density of the first medium to 1.08 g/mL to 1.10 g/mL and the density of the second medium to 1.06 g/mL to 1.08 g/mL. More preferably, the density of the first medium is 1.08 g/mL to 1.09 g/mL and the density of the second medium is 1.065 g/mL to 1.08 g/mL. As a specific example, it is possible to separate plasma components, eosinophils, and monocytes from desired fractions to be collected, by setting the density of the first medium to 1.085 g/mL and the density of the second medium to 1.075 g/mL. In addition, it is also possible to partially separate red blood cells, neutrophils, and lymphocytes therefrom. In the present invention, the types of the first medium and the second medium may be the same as or different from as each other, and are not limited as long as it is possible to realize the effect of the invention. However, use of the same types of media is a preferred aspect.

As the first medium and the second medium used in the concentration step, it is preferable to use media such as Percoll (registered trademark) which is a silica colloid particle dispersion liquid which has a diameter of 15 to 30 nm and is coated with polyvinyl pyrrolidone, Ficoll (registered trademark)-Paque which is a neutral hydrophilic polymer rich in a side chain made of sucrose, and Histopaque (registered trademark) which is a solution made of polysucrose and sodium diatrizoate. In the present embodiment, it is possible to preferably use Percoll (registered trademark) and Histopaque (registered trademark). A product of Percoll (registered trademark) having a density (specific gravity) of 1.130 is commercially available and it is possible to prepare a medium having a target density (specific gravity) by diluting the product. It is possible to prepare a first medium and a second medium to have a desired density using a medium having a density of 1.077 and a medium having a density of 1.119 as commercially available Histopaque (registered trademark).

[Other Concentration Methods]

As a method for concentrating nucleated red blood cells, it is possible to concentrate nucleated red blood cells by separating white blood cells from the nucleated red blood cells using an antibody specifically bound to CD45 (CD: cluster of differentiation) (a classification number of a monoclonal antibody based on CD classification) which is an antigen of a usual white blood cell, using a magnetic effect, in addition to the above-described density gradient centrifugation.

<Definition Step (Step S16)>

The definition step is a step of defining that a candidate cell of a nucleated red blood cell in accordance with the shape of a cell, from maternal blood in which nucleated red blood cells are concentrated through the concentration step.

Other blood components, such as white blood cells, other than the nucleated red blood cells are also contained in peripheral blood containing the nucleated red blood cells concentrated in the concentration step. The other blood cells can be removed from maternal blood through the concentration step. However, it is difficult to completely remove the other blood cells therefrom. In addition, a white blood cell of a single nucleus can be removed using Formula (1) and (2) shown below. However, it is also difficult to completely remove this white blood cell. Accordingly, gene analysis of a cell which has been sorted out as a nucleated red blood cell is performed. However, it is necessary to start again from production of a specimen for analysis, which results in inefficient analysis. It is possible to efficiently perform subsequent analysis while preventing such a case by defining that a candidate cell is a nucleated red blood cell through the definition step.

Fig. 2 is a flow chart showing a procedure of a definition step. The definition step includes a candidate cell selection step (step S32) of selecting a candidate cell of nucleated red blood cells in accordance with the shapes of cells; a white blood cell selection step (step S34) of selecting a white blood cell which becomes a standard in a case of defining the candidate cell as a nucleated red blood cell in a next nucleated red blood cell definition step; and the nucleated red blood cell definition step (step S36) of defining that the candidate cell is a nucleated red blood cell through comparison of spectral characteristics between the candidate cell and the white blood cell.

The definition step is performed using a specimen for analysis which has been produced by coating a transparent substrate, preferably slide glass, with peripheral blood containing the nucleated red blood cells concentrated in the concentration step.

(Sorting Using Information of Shape of Cell)

<<Candidate Cell Selection Step (Step S32)>>

In the definition step, a candidate cell of a nucleated red blood cell which becomes a candidate of a nucleated red blood cell is sorted out in accordance with information of the shape of cell (hereinafter, also referred to as the "shape of cell") using the above-described specimen for analysis.

Examples of the sorting performed in accordance with the shape of cell include the ratio of the area of a nuclear region to the area of cytoplasm, the degree of circularity of a nucleus, the area of a nuclear region, the brightness of a nucleus, and a crushed condition of a nucleus. Among these, it is preferable to sort a cell, which has conditions satisfying the ratio of the area of a nuclear region to the area of cytoplasm and the degree of circularity of a nucleus, as a candidate of fetus-derived nucleated red blood cell. The area of cytoplasm and the area of a nuclear region are areas on a face on which the specimen for analysis is observed.

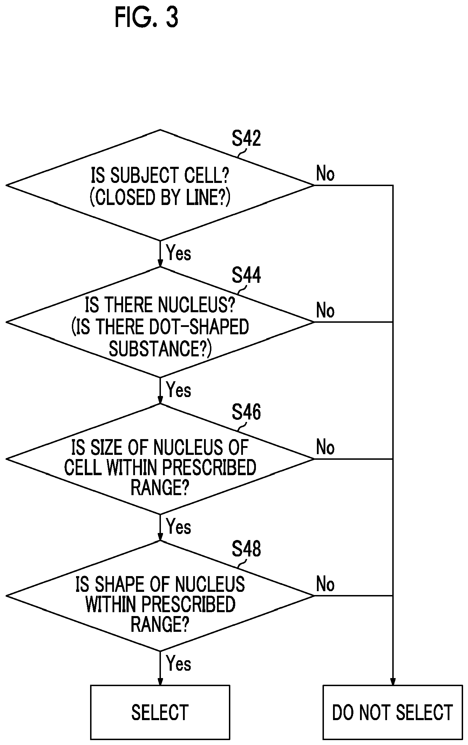

Fig. 3 is a flow chart showing a procedure of an example of a candidate cell selection step of performing sorting in accordance with the shape (shape of a nucleus) of a cell in the definition step.

In the sorting performed in accordance with the shape of a cell, cells are first extracted (Step S42). Determination of whether or not a subject is a cell is performed using a binary image. A binary image is generated using brightness information of an image which has been obtained by imaging a specimen for analysis with a white light source, as a predetermined threshold value, and a subject having dot-shaped and island-shaped continuous regions, that is, regions closed by an arbitrary closed curve are extracted as cells from the generated binarized image. The size of the binarized image may be obtained after arranging the shape of the continuous regions by performing morphology processing such as erosion and dilation.

Next, the cells extracted in Step S42 are sorted out depending on the presence or absence of a nucleus (Step S44). The presence or absence of a nucleus is determined depending on whether or not there is a nucleus (dot-shaped substance) in a cell. It is also possible to use the binary image in a case of determining the presence or absence of a nucleus. In a case where there is a region, which is closed by a closed curve and has an area smaller than that of the region extracted as a cell, in the region extracted as a cell, it is possible to extract the region as a nucleus.

Next, cells having 1 to 5 $\mu$m of a size (the size of dot-shaped and island-shaped continuous regions) of a nucleus are extracted (Step S46). The selection of cells having a nucleus with a size of 1 to 5 $\mu$m is effective for sorting nucleated red blood cells. The closed curve indicating the outer shape of a nucleus can be approximated to a circular shape or an elliptical shape and the "size of the dot-shaped and island-shaped continuous regions" can be set as a diameter of the circular shape, to which the outer shape of a nucleus is approximated, or a major axis of the elliptical shape, to which the outer shape of a nucleus is approximated.

Examples of the circular shape to which the outer shape of a nucleus is approximated include a circular shape having a minimum diameter, out of circular shapes circumscribing the closed curve indicating the outer shape of a nucleus. Examples of the elliptical shape to which the outer shape of a nucleus is approximated include an elliptical shape having a minimum major axis, out of elliptical shapes circumscribing the closed curve indicating the outer shape of a nucleus. Cells having 1 to 5 $\mu$m of a diameter of the circular shape to which the outer shape of a nucleus is approximated or a major axis of the elliptical shape to which the outer shape of a nucleus is approximated are extracted.

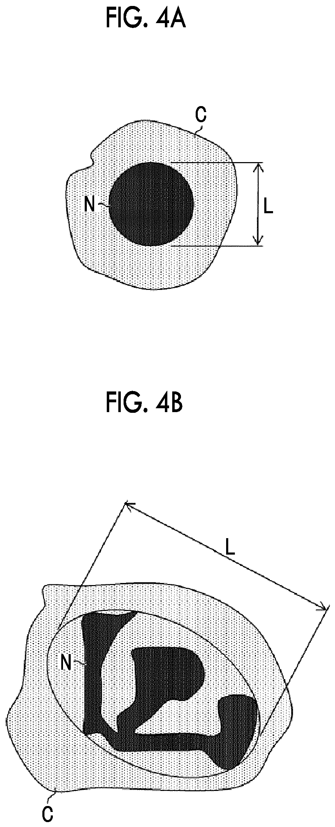

Next, cells are sorted in accordance with the shape of a nucleus (Step S48). Fig. 4A is a schematic view showing the shape of a cell to be selected, and Fig. 4B is a schematic view showing the shape of a cell to be removed. A method including a first step of performing sorting in accordance with the ratio of the area of a nuclear region to the area of cytoplasm and a second step of performing sorting in accordance with the degree of circularity of a nucleus is described as a specific example of sorting performed in accordance with the shape of a nucleus. In the first step, in a case where the ratio of the area of a nuclear region to the area of cytoplasm satisfies the following Formula (1), a cell is selected as a candidate cell of a nucleated red blood cell. In a case where the area of cytoplasm of a cell is set to C and the area of a nucleus of the cell is set to N, a cell having a nucleus of which the area of N to C exceeds 0.25 and less than 1.0 is selected as a candidate cell of a nucleated red blood cell.

$$0.25 < (N / C) < 1.0 \qquad (1)$$

In addition, examples of the second step satisfying the selection conditions of the degree of circularity of a nucleus include a case of satisfying the following Formula (2). In a case where the diameter of a nucleus or the major axis of a nucleus is set to L, a cell having a nucleus of which the ratio of the area of the nucleus to the square of the diameter of the nucleus or the major axis of the nucleus exceeds 0.65 and less than 0.785 is selected as a candidate cell of a nucleated red blood cell. The diameter of the nucleus or the major axis of the nucleus can be obtained through the same method as that in the case where the size of a nucleus is obtained in Step S44.

$$0.65 < (N / (L \times L)) < 0.785 \qquad (2)$$

In the sorting performed in accordance with the shape of a cell, it is possible to reduce the number of candidate cells on which sorting performed through image processing is to be performed, by performing rough sieving in the first sorting step in accordance with Step S42 to Step S46 and performing sorting through the shape of a nucleus in the second step in accordance with Step S48, and therefore, it is possible to efficiently sort out candidate cells.

In the sorting performed in accordance with the shape, it is necessary to satisfy all of the conditions of Step S42 to Step S48. Cells which do not satisfy any one of the conditions of Step S42 to Step S48 are not selected as candidate cells.

Configuration examples of a system of sorting out nucleated red blood cells which become candidates of fetus-derived nucleated red blood cells using the information of the shape of a cell include a system equipped with an optical microscope, a digital camera, a stage for slide glass, an optical transfer system, an image processing personal computer (PC), a control PC, and a display. The optical transfer system includes an objective lens and a charge coupled device (CCD) camera. Examples of the image processing PC include a configuration including a processing system of performing analysis and storing data. Examples of the control PC include a configuration of including a control system of controlling the position of storage for slide glass or controlling the entire processing.

(Sorting Performed in accordance with Optical Characteristics)

In a case of performing the sorting in accordance with optical characteristics, it is possible to use spectral characteristics attributable to hemoglobin contained in red blood cells. It is defined that the candidate cell of a nucleated red blood cell selected in the candidate cell selection step is a nucleated red blood cell, using the difference in spectral characteristics between the candidate cell and white blood cells existing around this candidate cell. For example, whether the candidate cell is a nucleated red blood cell is defined using the difference in spectral characteristics between the candidate cell of a nucleated red blood cell and white blood cells existing in the vicinity of this candidate cell, using blood cells with which a glass substrate is coated.

The presence or absence of hemoglobin is different between the nucleated red blood cell and the white blood cell which is one type of nucleated cell in blood. An aspect in which the optical information is information attributable to hemoglobin is preferable. Moreover, it is preferable that the optical information is information attributable to the difference in absorbances in at least one monochromatic light beam selected from wavelengths included in a light wavelength region of 400 to 650 nm. Since the nucleated red blood cell has hemoglobin, the nucleated red blood cell exhibits a high absorption coefficient in light wavelength regions of 400 to 650 nm. Since the white blood cell does not have hemoglobin, the absorption coefficient is decreased in these wavelength regions. Here, the absorption coefficient indicates a constant showing that how much light is absorbed by a certain medium on which light is incident.

It is more preferable that the light wavelength region is a light wavelength region of 400 to 450 nm or a light wavelength region of 525 to 580 nm. Absorption coefficients in these light wavelength regions show high value in a nucleated red blood cell since there is hemoglobin, and therefore, it is possible to make the difference between the absorption coefficients of the nucleated red blood cell and the absorption coefficients of cytoplasm of a white blood cell large. Accordingly, it is possible to clarify the difference between the nucleated red blood cell and the white blood cell and to accurately determine whether a cell is a nucleated red blood cell.

In addition, it is possible to discriminate nucleated red blood cells after measuring absorbances at a plurality of wavelengths in each of the light wavelength regions and using an average value of these absorbances. It is possible to reduce an influence of noise included in the measurement values of the absorbances, by performing the sorting in accordance with the average value of the absorbances.

As a method for defining that the candidate cell is a nucleated red blood cell through spectral characteristics, whether a candidate cell is a nucleated red blood cell is determined by comparing the candidate cell with white blood cells existing around the candidate cell of the nucleated red blood cell. For example, the ratio of the absorbance of a candidate cell to the absorbance (absorbance of candidate cell / absorbance of white blood cell) of a white blood cell is obtained after measuring the absorbance of the white blood cell at a monochromatic light at the same wavelength as that of the candidate cell of the nucleated red blood cell. In a case where the candidate cell is a nucleated red blood cell, a high absorbance is exhibited. Therefore, the proportion of the absorbances becomes larger than "1". In addition, in a case where the candidate cell is a white blood cell, the absorbance of the candidate cell becomes almost equal to that of the white blood cell. Therefore, the proportion of the absorbances becomes close to "1". Accordingly, by performing gene analysis after prioritizing the possibility of being a nucleated red blood cell from a cell having a high value after measuring the proportions of the absorbances in a plurality of candidate cells, it is possible to increase the possibility that the candidate cells on which the gene analysis is performed may be nucleated red blood cells.

«White Blood Cell Selection Step (Step S34): Method for Selecting White Blood Cells»

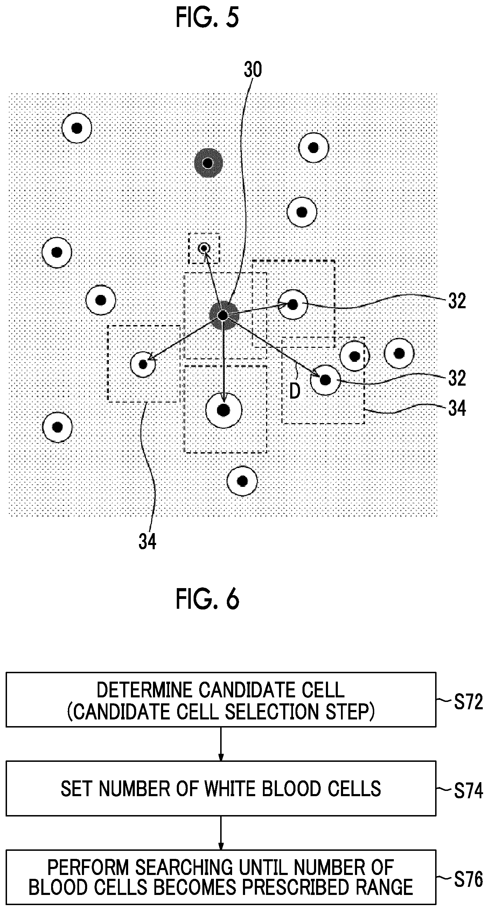

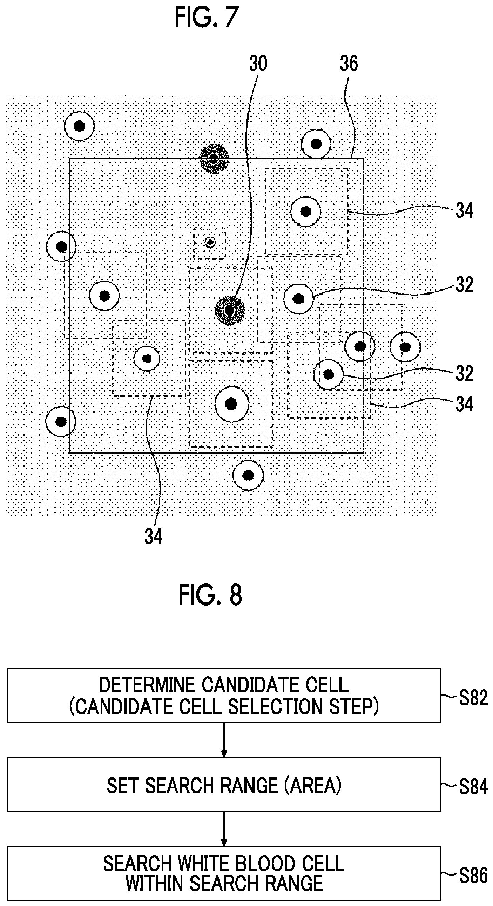

Selection of white blood cells can be performed through the following method. Figs. 5 to 8 are views illustrating methods for selecting white blood cells. Fig. 5 is a view illustrating a method for setting a search range based on the number of white blood cells and Fig. 6 is a flow chart showing a procedure of setting the search range based on the number of white blood cells. Fig. 7 is a view illustrating a method for setting a search range of white blood cells based on the area of a candidate cell and Fig. 8 is a flow chart showing a procedure of setting the search range of white blood cells based on the area of the candidate cell.

{Method for Setting Search Range Based on Number of White Blood Cells}

Figs. 5 and 6 are views illustrating a method for setting a search range based on the number of white blood cells. First, a candidate cell 30 of a nucleated red blood cell is determined (Step S72 in Fig. 6). The candidate cell 30 of the nucleated red blood cell can be determined through the candidate cell selection step as described above. Next, the number of white blood cells to be searched is set (Step S74 in Fig. 6). A case in which the number of white blood cells to be searched is set to 5 is described in Fig. 5. Next, white blood cells 32 are selected until the number of white blood cells to be searched becomes a set number around the candidate cell 30 (Step S76 in Fig. 6). The white blood cells 32 are selected in an ascending order of a distance D from the center of the candidate cell 30 to the center of a white blood cell 32.

The center of the candidate cell 30 can be set as the center of a circular shape in a case where the planar shape of the candidate cell 30 is approximated to the circular shape. Similarly, the center of the white blood cell 32 can be set as the center of a circular shape in a case where the planar shape of the white blood cell 32 is approximated to the circular shape. The same principle also applies to the following description. In addition, white blood cells may be selected in an ascending order of the distance from the center of gravity of the candidate cell 30 to the center of gravity of the white blood cell 32.

In Fig. 5, it has been described that the number of white blood cells selected is 5. However, the number of white blood cells selected is preferably 2 to 20. A standard which is different from the candidate cell can be determined without variation by setting the number of white blood cells selected to be within the above-described range and obtaining and averaging optical information of the plurality of white blood cells. In addition, the number of white blood cells selected is more preferably set to be 3 to 10. A large number of white blood cells are preferable since it is possible to average optical information as the number of white blood cells is larger. However, it takes time to measure the white blood cells if the number of white blood cells is large, and therefore, it is preferable that the number of white blood cells is within the above-described range.

A cell which has a nucleus but does not satisfy the above-described Formulas (1) and (2) is selected as a white blood cell 32 to be selected, in the candidate cell selection step. There are nucleated red blood cells and white blood cells as cells having a nucleus in blood. By selecting a cell which does not satisfy Formulas (1) and (2), it is possible to increase a possibility that the candidate cell is a white blood cell.

{Method for Setting Search Range Based on Area of Candidate Cell}

Figs. 7 and 8 are views for illustrating a method for setting a search range based on the area of a candidate cell. Even in the method, first, a candidate cell 30 of a nucleated red blood cell is determined (Step S82 in Fig. 8) similarly to the method for setting a search range based on the number of white blood cells. Next, a search range 36 of the white blood cells 32 is set (Step S84 in Fig. 8). A search range 36 is shown by a solid line in Fig. 7. The search range 36 shown in Fig. 7 is set to 100 cell fractions (10 x 10 cell fractions) of the candidate cells 30.

It is preferable that the search range 36 is set to a square of which a side around the candidate cell 30 is 10 cell fractions of the candidate cell 30, or to a circular shape of which the diameter is 10 cell fractions. In addition, in a case where the search range 36 is set stepwise and there is no white blood cell in the search range 36, it is preferable to widen the search range 36. For example, in a case where the search range 36 is set to a square of which a side is 10 cell fractions of the candidate cell 30, or to a circular shape of which the diameter is 10 cell fractions, if there is no white blood cell 32, it is preferable to determine white blood cells after further widening the search range up to a square of which a side is 20 cell fractions or a circular shape of which the diameter is 20 cell fractions.

Next, white blood cells 32 within the search range 36 are searched and selected (Step S86 in Fig. 8). The standard to be selected as a white blood cell 32 is determined based on whether or not the center or the center of gravity of the white blood cell 32 is within the search range 36. In addition, regarding the white blood cells selected herein, a cell which has a nucleus but does not satisfy the above-described Formulas (1) and (2) is selected as a white blood cell 32 similarly to the case of setting a search range based on the number of white blood cells.

The number of white blood cells 32 selected and the area of the search range 36 are not particularly limited, and can be arbitrarily set.

In addition, as the method for selecting white blood cells, the method for selecting white blood cells after setting a search range based on the number of white blood cells selected shown in Figs. 5 and 6 and the method for selecting white blood cells after setting a search range based on the area of a candidate cell shown in Figs. 7 and 8 have been exemplified. However, the method for selecting white blood cells is not limited thereto, and it is also possible to perform the sorting through a method in which these methods are combined. For example, in a case where the number of white blood cells included in a search range which has been determined using the area of a candidate cell exceeds the number of white blood cells which has been previously set, it is possible to measure all white blood cells or to measure white blood cells after selecting the number of white blood cells which has been previously set. As the selection method, a white blood cell of which the distance between the center of a candidate cell and the center of the white blood cell is short or a white blood cell of which the distance between the center of gravity of a candidate cell and the center of gravity of the white blood cell is short is sequentially selected as described above. In addition, in a case where the number of white blood cells within the search range is smaller than the number of white blood cells which has been previously set, the search range is enlarged at a point in time at which the small number of white blood cells is determined, and the white blood cells included in the increased area due to the enlargement of the search range are detected. It is preferable to repeat this operation until a sufficient number of white blood cells which has been previously determined is obtained.

<<Nucleated Red Blood Cell Definition Step (Step S36)>>

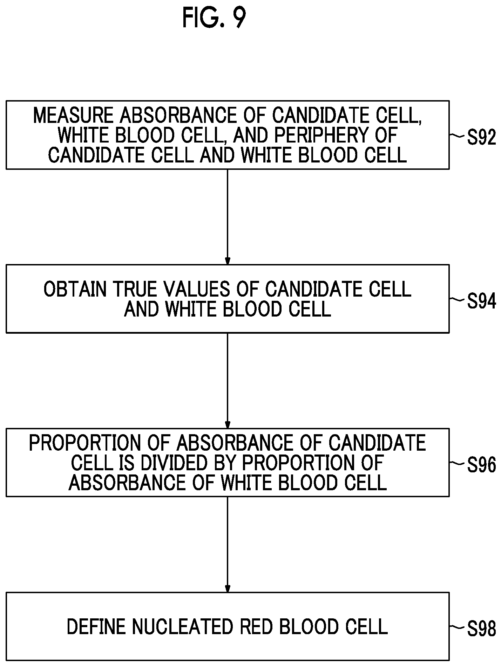

Fig. 9 is a flow chart showing a procedure of a nucleated red blood cell definition step.

After selecting white blood cells 32, absorbances of (1) a candidate cell 30 of a nucleated red blood cell, (2) white blood cells 32, and (3) a periphery 34 (shown by a broken line in Figs. 5 and 7) of the candidate cell 30 and the white blood cells 32 are measured at selected wavelengths (Step S92 in Fig. 9). The periphery 34 of the candidate cell 30 and the white blood cells 32 refers to a range of 9 cell fractions (3 x 3 cell fractions) of a target candidate cell 30 and target white blood cells 32 in Figs. 5 and 7. Since there are white blood cells also within the periphery 34 of the candidate cell 30 and the white blood cells 32, the absorbances of regions other than white blood are measured within the periphery 34 during the measurement of the absorbances of the periphery 34.

After measuring the absorbances, (4) true values of the absorbances of the candidate cell 30 are obtained by subtracting an absorbance of the periphery 34 of the candidate cell 30 from the absorbance of the candidate cell 30. In addition, (5) true values of the absorbances of the white blood cells are obtained by subtracting an absorbance of a periphery 34 of each white blood cell from the absorbances of the white blood cells 32 (Step S94).

Next, the ratio of the absorbance of the candidate cell 30 to the absorbances of the white blood cells 32 is obtained (Step S96). In a case of selecting a plurality of white blood cells 32, the absorbance of a white blood cell 32 is set by an average value of the absorbances of white blood cells. Since there is no hemoglobin in white blood cells, in a case where the candidate cell 30 is a nucleated red blood cell, the absorbance of the candidate cell 30 becomes higher than that of the white blood cell 32 and the proportion of the absorbances becomes larger than "1". In addition, in a case where the candidate cell 30 is a white blood cell, the absorbance of the candidate cell 30 is almost equal to that of the white blood cell 32. Therefore, the proportion of the absorbances exhibits a numerical value close to "1". Accordingly, by obtaining the proportion of the absorbances, it is possible to determine whether the candidate cell 30 is a nucleated red blood cell and to define that the candidate cell is a nucleated red blood cell (Step S98).

In addition, it is possible to perform prioritization that there is high possibility that a candidate cell of which the proportion of the absorbances is larger than "1" is a nucleated red blood cell, after obtaining the proportion of the absorbances. It is possible to efficiently perform gene analysis and definition of a nucleated red blood cell by checking gene information from the candidate cell 30 with a high priority.

In addition, a method for determining whether the candidate cell 30 is a nucleated red blood cell after obtaining the proportion (ratio) of the absorbance of the candidate cell 30 to the absorbances of the white blood cells 32 has been described above. However, it is also possible to determine a nucleated red blood cell after taking the difference between the absorbance of the candidate cell 30 and the absorbance of the white blood cells. In this case, it is possible to prioritize that there is a high possibility that a candidate cell 30 which has a large absolute value of a numerical value obtained by taking the difference between the absorbance of the candidate cell 30 and the absorbance of white blood cells may be a nucleated red blood cell, and there is a high possibility that a candidate cell 30 which has a small absolute value of a numerical value obtained by taking the difference between the absorbance of the candidate cell 30 and the absorbance of white blood cells may be a white blood cell.

It is possible to select a candidate cell and define that this candidate cell is a nucleatedred blood cell, through the above method.

<Amplification Step (Step S18)>

The amplification step is a step of amplifying nucleic acids contained in chromosomes of cells which have been defined as nucleated red blood cells through the definition step. In addition, in a case where whether a nucleated red blood cell is a fetus-derived nucleated red blood cell or a mother-derived nucleated red blood cell is determined by comparing alleles with each other in the next sorting step (Step S20), it is preferable to perform amplification of nucleic acids contained in chromosomes on white blood cells which have not been selected in the candidate cell selection step or white blood cells which have been selected in the white blood cell selection step.

The cells which have been defined as nucleated red blood cells through the definition step are separated from a specimen using a micromanipulator. DNA is extracted from the cells which have been obtained by being separated from the specimen, and whole genome amplification is performed. The whole genome amplification can be performed using a commercially available kit.

Genome DNA obtained through elution from the obtained cells through cytolysis using a surfactant and a protein decomposition step using protease K or the like which are general methods is used for the whole genome amplification used in the present embodiment.

It is possible to use a reagent Single cell WGA kit (New England Biolabs) based on a polymerase chain reaction (PCR), a GenomePlex Single Cell Whole Genome Amplification kit (Sigma-Aldrich Co. LLC.), and Multiple Annealing and Looping-Based Amplification Cycles (MALBAC method) (published in WO2012/166425A2) as a whole genome amplification reagent. In addition, it is possible to similarly use a reagent GenomiPhi (GE Healthcare) and REPLI-g (QIAGEN) based on a chain-substituting DNA synthesis reaction. In the present embodiment, it is preferable to use a Single cell WGA kit (New England Biolabs).

It is preferable to check whether or not amplification is performed on an amplified product of DNA which has been obtained through whole genome amplification, through agarose gel cataphoresis. Furthermore, it is preferable to purify the whole genome amplification product using QIAquick PCR Purification Kit (QIAGEN).

In addition, it is preferable to measure the concentration of the amplified product of DNA which has been obtained through whole genome amplification using NANODROP (Thermo Fisher Scientific Inc.) and BIOANALYZER (Agilent Technologies).

In the amplification step, it is preferable to amplify nucleic acids obtained from a plurality of cells which have been defined as nucleated red blood cells in the definition step. In the present embodiment, it is necessary to sort out fetus-derived nucleated red blood cells since it is assumed that a prenatal diagnosis is applied. After definition step, it is unclear whether nucleated red blood cells are fetus-derived nucleated red blood cells or mother-derived nucleated red blood cells. Therefore, it is preferable to amplify the nucleic acids obtained from a plurality of nucleated red blood cells and perform sorting in the next sorting step.

<Sorting Step (Step S20)>

The sorting step is a step of sorting the nucleated red blood cells, which have been defined through the definition step, into fetus-derived nucleated red blood cells and mother-derived nucleated red blood cells.

(Gene Analysis)

[Analysis Using Allele]

After performing whole genome amplification through the amplification step, it is possible to determine whether the nucleated red blood cells are fetus-derived nucleated red blood cells or mother-derived nucleated red blood cells by determining a sequence of an allele.

The amount of an amplified product of DNA which has been defined as a nucleated red blood cell and has been amplified through a polymerase chain reaction, and has a sequence of a region of 100 to 150 base pair (bp) which has been previously determined, with respect to a chromosome becoming a subject for which it is inspected whether it is a fetus-derived nucleated red blood cell or a mother-derived nucleated red blood cell, is obtained using a sequencer. In the present embodiment, it is preferable that a chromosome which is to be inspected is chromosome 13. A fetus-derived nucleated red blood cell usually inherits a set of chromosomes from each of a father and a mother, and therefore, has two chromosomes from a father and two chromosomes from a mother excluding sex chromosomes. It is possible to determine whether a nucleated red blood cell is a fetus-derived nucleated red blood cell or a mother-derived nucleated red blood cell by checking the presence of a fathered gene after analyzing alleles of each set of chromosomes thereof.

Checking of the presence of a father-derived gene is performed such that, in a case where there is an allele which does not exist in a mother-derived cell after similarly performing gene analysis also on a mother-derived cell, it is possible to recognize that this allele is a father-derived gene. In a case of recognition of a father-derived gene, it is possible to determine that the nucleated red blood cell is a fetus-derived nucleated red blood cell. DNA analysis of a mother-derived cell is performed using a white blood cell which has not been selected in the candidate cell selection step or a white blood cell which exists in a specimen (on slide glass) and has been selected in the white blood cell selection step.

In an allele to be analyzed, it is preferable to analyze single nucleotide polymorphism (SNP (SNPs)) or short tandem repeat (STR) sequence.

A gene of a fetus which is inherited from each pair of genes from parents, and genetic information is recorded in a sequence of a chemical substance of 4 types of bases. In a case of humans, there are about three billion bases, and there are different sequence parts depending on individuals at a ratio of one base to 1000 to 2000 bases. This is called single nucleotide polymorphism. If it is possible to check a sequence of single nucleotide polymorphism in a nucleated red blood cell by comparing this single nucleotide polymorphism, which has been analyzed, with a white blood cell which is a mother-derived cell, it is possible to determine that the nucleated red blood cell is derived from a fetus.

A short tandem repeat sequence is a repeating region in which DNA sequences, one of which is a unit, are repeatedly arranged in series in DNA. Since a fetus inherits a short tandem repeat sequence from a father and a mother, it is possible to determine that the nucleated red blood cell, which has a short tandem repeat sequence different from that of a mother-derived white blood cell, is a fetus-derived nucleated red blood cell.

[Analysis Using Y Chromosome]

In a case where a fetus is a male infant, it is possible to determine whether a nucleated red blood cell is a fetus-derived nucleated red blood cell or a mother-derived nucleated red blood cell by checking the presence or absence of a Y chromosome after performing whole genome amplification through the amplification step.

Since a Y chromosome exists only in a male, there is no Y chromosome in a mother-derived nucleated red blood cell. Accordingly, in a case where a fetus is a male infant, it is possible to sort a nucleated red blood cell into a fetus-derived nucleated red blood cell if it is possible to check the presence of a Y chromosome.

<Determination Step (Step S22)>

The determination step is a step of determining the presence or absence of a numerical abnormality of the fetus-derived chromosome through comparison of the amounts of amplified products of DNAs of the fetus-derived nucleated red blood cells sorted out through the sorting step.

A chromosome other than a chromosome for which a numerical abnormality is to be inspected is selected as a standard (or a reference) for determining the presence or absence of a numerical abnormality of a fetus-derived chromosome, and the amplification amount of an amplified product of DNA having a sequence of a region of 100 to 150 bp which has been previously determined is obtained using a sequencer. As a chromosome (standard chromosome) which becomes a standard, an aspect, in which at least one chromosome other than a chromosome for which a numerical abnormality of chromosomes of fetus-derived nucleated red blood cells is to be inspected is selected, or an aspect, in which a chromosome existing in a cell which is identified as a mother-derived nucleated red blood cell is selected, is selected. In the present embodiment, it is preferable to select a chromosome existing in a cell which is identified as a mother-derived nucleated red blood cell.

Next, whether or not there is a numerical abnormality in a fetus-derived chromosome is determined from the ratio of the amount of an amplified product of DNA of a target chromosome of an inspection of a numerical abnormality to the amount of an amplified product of DNA of a reference chromosome. In a case where a fetus is in a normal condition, it is expected that the ratio of the amount of an amplified product of DNA of a fetus-derived chromosome for which a numerical abnormality is to be inspected to the amount of an amplified product of DNA of a reference chromosome may be approximately 1:1. In a case of trisomy which is a numerical abnormality in which there are three chromosomes whereas there are two chromosomes in a normal case, it is expected that the ratio may be 1.0:1.5 (or 2:3).

In addition, before the determination step, a distribution of results in which the ratios of the amounts of amplified products of DNAs of fetus-derived chromosomes to the amounts of amplified products of DNAs of mother-derived chromosomes, which have been collected from a plurality of pregnant mothers, in a case where each mother is pregnant with a normal fetus are obtained in plural times, and a distribution of results in which the ratios of the amounts of amplified products of fetus-derived DNAs to the amounts of amplified products of mother-derived DNAs of each mother who pregnant with a fetus with trisomy are obtained in plural times are previously obtained, and a cutoff value is set in a region where these two distributions do not overlap. Then, it is possible to determine whether or not there is a numerical abnormality by comparing the ratios of the amounts of the amplified products of DNAs with this cutoff value. In this case, it is possible to interpret the inspection results such that a fetus is normal if the ratio of the amount of an amplified product of DNA is less than or equal to the cutoff value, and there is a numerical abnormality which is trisomy if the ratio thereof is greater than or equal to the cutoff value.

[Example 1]

<Example 1>

Hereinafter, the present invention will be described in more detail using examples exemplified below. However, the present invention is not limited to these examples.

(Collection Step [Peripheral Blood Sample Acquisition Step])

After adding 10.5 mg of sodium salts of ethylenediaminetetraacetic acid (EDTA) to a 7 mL blood collecting tube as an anticoagulant, 7 mL of peripheral blood was obtained in the blood collecting tube as volunteer blood after obtaining informed consent from a volunteer of a mother. Thereafter, blood was diluted using a physiological salt solution.

(Concentration Step [Concentration Step of Nucleated Red Blood Cell])

A liquid with a density of 1.070 g/mL and a liquid with a density of 1.095 g/mL were prepared using a PERCOLL liquid (registered trademark), and 2 ml of a liquid with a density of 1.095 g/mL was added to a bottom portion of a centrifuge tube. Continuously, 2 mL of a liquid with a density of 1.070 g/mL was made to slowly overlap the top of the liquid with a density of 1.095 g/mL so as not to disturb an interface. Thereafter, 11 mL of diluent of blood which had been collected in the above was slowly added to the top of the medium with a density of 1.070 g/mL in the centrifuge tube. Then, centrifugation was performed for 20 minutes at 2000 revolution per minute (rpm). Thereafter, the centrifuge tube was taken out and fractions which had been deposited between the solution with a density of 1.070 g/mL and the solution with a density of 1.090 g/mL were collected using a pipette. A first glass substrate was held by one hand and a drop of the fractions of blood which had been collected in this manner was placed at one end of the first glass substrate. Another glass substrate (second glass substrate) was held by the other hand and one end of the second glass substrate was brought into contact with the first glass substrate at 30 degrees. If the lower surface of the second glass substrate which is brought into contact with the fractions of blood, the fractions of blood spread into the space surrounded by the two sheets of glass due to a capillary phenomenon. Next, the first glass substrate was coated with the fractions of blood and the second glass substrate was made to be slid in a direction of a region opposite to the region of the first glass substrate, on which blood was placed, while maintaining the angle. Thereafter, the first glass substrate was sufficiently dried through air blowing for one hour. This first glass substrate was immersed in a MAY-Grunwald dyeing liquid for three minutes and was washed by being immersed in a phosphoric acid buffer solution. Thereafter, the first glass substrate was immersed in a GIEMSA dyeing liquid (which was diluted with a phosphoric acid buffer solution to make a concentration of 3%) for 10 minutes. Thereafter, the first glass substrate was dried after being washed with pure water. A plurality of glass substrates dyed in this manner were produced.

(Definition Step)

<Candidate Cell Selection Step [Sorting Step Based on Shape of Cell]>

In order to sort out a nucleated red blood cell which becomes a candidate of a fetus-derived nucleated red blood cell, from blood after being subjected to concentration with which the glass substrate was coated, an electric two-dimensional stage (hereinafter, denoted as an XY stage), a measurement system of an optical microscope including an objective lens and a CCD camera, a control unit including an XY stage control unit and a Z-direction control unit of controlling a vertical direction (hereinafter, denoted as a Z-direction) movement mechanism, and a control unit portion including an image input unit, an image processing unit, and an XY position recording unit. Blood cells which had been prepared as described above and with which the glass substrate was coated were placed on the XY stage and scanning was performed by being focused on the glass substrate, an image which had been obtained using an optical microscope was taken, and nucleated red blood cells which were target cells were searched through image analysis.

In the image analysis, the positions of cells in an X-direction and a Y-direction of the detected cells were recorded in a case where cells which satisfied the conditions of the following Formulas (1) and (2) were detected, a coordinate system consisting of two axes orthogonal to an image to be analyzed was set, and one axis of the two axes was set to an X-axis and the other of the two axes was set to a Y axis.

$$0.25 < (N / C) < 1.0 \qquad (1)$$

$$0.65 < (N / (L \times L)) < 0.785 \qquad (2)$$

Here, C is defined as the area (the area of a region indicating cytoplasm in an image used for analysis) of cytoplasm of a cell for which image analysis is performed, N is defined as the area (the area of a region indicating a nucleus of a cell in an analysis image) of a nucleus of a cell for which image analysis is performed, L is defined as the major axis or the diameter (the major axis of an elliptical shape in a case where a region indicating a nucleus of a cell in an analysis image is approximated to the elliptical shape, or the diameter of a circular shape in a case where a region indicating a nucleus in a cell in an analysis image is approximated to the circular shape) of a nucleus of a cell for which image analysis is performed, that is, the major axis of an elliptical shape or the diameter of a circular shape circumscribing a cell nucleus which has a complicated shape. A candidate cell which became a candidate of a fetus-derived nucleated red blood cell existing on a slide glass substrate satisfying (1) and (2) was selected.

<White Blood Cell Selection Step, Nucleated Red Blood Cell Definition Step [Step of Sorting Nucleated Red Blood Cell Based on Optical Characteristics]>

Analysis of optical characteristics was performed on ten candidate cells which had been selected according to information of the shape using a microspectroscopic device. Ten candidate cells of nucleated red blood cells on a slide glass substrate were specified, one nucleated red blood cell out of the ten candidate cells was irradiated with monochromatic light having a wavelength of 415 nm, and an absorbance 1 was measured. Next, with respect to white blood cells which were at a position in the vicinity of the candidate cells and of which the shape of a nucleus does not satisfy the above-described Formula (2), five white blood cells were selected in an ascending order of the distance from the candidate cells, each white blood cell was similarly irradiated with monochromatic light having a wavelength of 415 nm, the absorbances were measured, and an average thereof was obtained as an absorbance 2.

Measurement of the absorbance 1 was similarly performed on remaining nine cells of the candidate cells on the glass substrate through the same method. The absorbances of the five white blood cells which were at a position in the vicinity of the candidate cells were respectively measured. An average value thereof was calculated and was set as the absorbance 2.

The absorbance 1 with respect to the absorbance 2 (absorbance 1/absorbance 2) was obtained from this calculation result, and cells of which the proportions of the absorbances became greater than or equal to 1 were extracted. As a result, six cells of which the proportions of the absorbances were obviously greater than or equal to 1 were detected. Since there is no hemoglobin in white blood cells, in a case where a candidate cell is a red blood cell, the numerical value becomes high after obtaining the proportions of the absorbances. Accordingly, it is possible to define that a candidate cell of which the value is obviously greater than or equal to 1 is a nucleated red blood cell after obtaining the proportions of the absorbances.

(Cell Isolation Step)

Six cells which had been detected in the above-described step and of which the proportions of the absorbances became greater than or equal to 1 were collected using a micromanipulator.

(Amplification Step [DNA Amplification Step])

Whole genome amplification was performed on the six cells, which had been collected using the micromanipulator, using Single Cell WGA kit manufactured by New England Biolabs to amplify a small amount of DNA in the cells into about a million times in accordance with description of a manual.

(Sorting Step [Step of Defining Whether Cell Is Derived from Mother or Fetus])

It was possible to confirm that there is SNP information in one cell, out of six cells, which is different from the others through comparison of SNP (SNP ID: rs3751355, rs1799955, rs2297555, and rs9571740) of a C1QTNF9B gene region, a PCDH9 gene region, a BRCA2 gene region, and a MTRF1 gene region of chromosome 13 with each other using a genome analyzer MISEQ manufactured by Illumina, Inc. after equally dividing the whole genome amplification product which had been amplified from each cell and using one of the divided whole genome amplification products. A cell which was selected in the white blood cell selection step was separately collected using a micromanipulator, and DNA was amplified similarly to six cells which were defined as nucleated red blood cells, to check SNP. As a result, it was confirmed that SNP of the one cell was coincident with SNP of the five cells. From the above, it was confirmed that the one cell was a fetus-derived nucleated red blood cell and the five cells were mother-derived nucleated red blood cells out of six cells which were defined as nucleated red blood cells.

<Example 2>

The five cells were collected in the nucleated red blood cell sorting step in the same manner as in Example 1 after peripheral blood from a mother different from the mother from which blood was collected in Example 1, and the same process was performed up to the amplification step [DNA amplification step]. Thereafter, the whole genome amplification product which had been amplified from each cell was equally divided and one of the divided whole genome amplification products was used to perform the sorting step.

(Sorting Step)

The presence or absence of a PCR amplified product of a Y chromosome was confirmed through a general agarose gel electrophoresis method by performing PCR using a Multiplex PCR ASSAY Kit manufactured by TAKARA BIO INC. using PCR primer which had been produced in a sex-determining factor (SRY) gene region of the Y chromosome. As a result, Y chromosome-derived information was detected with respect to one cell. It was confirmed that this cell was derived from a fetus and was a male infant. It was recognized that the other four cells were mother-derived nucleated red blood cells since no information derived from a Y chromosome was observed.

Explanation of References

30: candidate cell
32: white blood cell
34: periphery
36: search range

Claims

1. A method for sorting nucleated red blood cells, the method comprising:

a candidate cell selection step of selecting a candidate cell of nucleated red blood cells in accordance with shapes of cells;

a white blood cell selection step of setting a search range including the candidate cell and selecting a white blood cell within the search range;

a nucleated red blood cell definition step of defining that the candidate cell is a nucleated red blood cell through comparison of spectral characteristics of hemoglobin between the candidate cell and the white blood cell; and

a sorting step of sorting the nucleated red blood cells into mother-derived nucleated red blood cells or fetus-derived nucleated red blood cells through comparison of an allele contained in the nucleated red blood cell after the nucleated red blood cell definition step with an allele contained in a white blood cell which has not been selected in the candidate cell selection step or in the white blood cell selected in the white blood cell selection step.

2. A method for sorting nucleated red blood cells, the method comprising:

a candidate cell selection step of selecting a candidate cell of nucleated red blood cells in accordance with shapes of cells;

a white blood cell selection step of setting a search range including the candidate cell and selecting a white blood cell within the search range;

a nucleated red blood cell definition step of defining that the candidate cell is a nucleated red blood cell through comparison of spectral characteristics of hemoglobin between the candidate cell and the white blood cell; and

a sorting step of sorting the nucleated red blood cells into mother-derived nucleated red blood cells or fetus-derived nucleated red blood cells by checking presence or absence of a Y chromosome contained in the nucleated red blood cells after the nucleated red blood cell definition step.

3. The method for sorting nucleated red blood cells according to claim 1 or 2,
wherein the search range is a range which is set stepwise from the candidate cell based on a size of the candidate cell,
wherein the white blood cell within the search range is selected in the white blood cell selection step, and
wherein the search range is widened in accordance with the number of the selected white blood cells.

4. The method for sorting nucleated red blood cells according to claim 1 or 2,
wherein the search range is a range in which the white blood cell having a distance from the candidate cell is shortest and the white blood cell having a distance from the candidate cell becomes long are sequentially selected, and a number of the white blood cells which have been selected is 2 to 20.

5. The method for sorting nucleated red blood cells according to any one of claims 1 to 4,
wherein the spectral characteristics are absorbances in at least one monochromatic light beam selected from wavelengths included in a light wavelength region of 400 to 650 nm.

6. The method for sorting nucleated red blood cells according to any one of claims 1 to 5,
wherein the candidate cell selection step includes a first step of selecting a cell of which a ratio of an area of a cell nucleus of the cell to an area of cytoplasm of the cell satisfies Formula 1, in a case where the area of cytoplasm of the cell is set to C and the area of the cell nucleus of the cell is set to N.

$$0.25 < N / C < 1.0 \qquad \text{Formula 1}$$

7. The method for sorting nucleated red blood cells according to any one of claims 1 to 6,
wherein the candidate cell selection step includes a second step of selecting a cell of which a ratio of an area of a cell nucleus to a square of a length of a major axis of a circular shape or a major axis of an elliptical shape satisfies Formula 2, in a case where the area of the cell nucleus of the cell is set to N and the length of the diameter of the circular shape circumscribing the cell nucleus of the cell or the major axis of the elliptical shape is set to L.

$$0.65 < N / (L \times L) < 0.785 \qquad \text{Formula 2}$$

**Amended claims under Art. 19.1 PCT**

1. (Amended) A method for sorting nucleated red blood cells, the method comprising:

a candidate cell selection step of selecting a candidate cell of nucleated red blood cells in accordance with shapes of cells;
a white blood cell selection step of setting a search range including the candidate cell and selecting a white blood cell within the search range;
a nucleated red blood cell definition step of defining that the candidate cell is a nucleated red blood cell through comparison of spectral characteristics of hemoglobin between the candidate cell and the white blood cell; and
a sorting step of sorting the nucleated red blood cells into mother-derived nucleated red blood cells or fetus-derived nucleated red blood cells through comparison of an allele contained in the nucleated red blood cell after the nucleated red blood cell definition step with an allele contained in a white blood cell which has not been selected in the candidate cell selection step or in the white blood cell selected in the white blood cell selection step,
wherein the search range is a range which is set stepwise from the candidate cell based on a size of the candidate cell,
wherein the white blood cell within the search range is selected in the white blood cell selection step, and
wherein the search range is widened in accordance with the number of the selected white blood cells.

2. (Amended) A method for sorting nucleated red blood cells, the method comprising:

a candidate cell selection step of selecting a candidate cell of nucleated red blood cells in accordance with shapes of cells;
a white blood cell selection step of setting a search range including the candidate cell and selecting a white blood cell within the search range;
a nucleated red blood cell definition step of defining that the candidate cell is a nucleated red blood cell through comparison of spectral characteristics of hemoglobin between the candidate cell and the white blood cell; and
a sorting step of sorting the nucleated red blood cells into mother-derived nucleated red blood cells or fetus-derived nucleated red blood cells by checking presence or absence of a Y chromosome contained in the nucleated red blood cells after the nucleated red blood cell definition step,
wherein the search range is a range which is set stepwise from the candidate cell based on a size of the candidate cell,
wherein the white blood cell within the search range is selected in the white blood cell selection step, and
wherein the search range is widened in accordance with the number of the selected white blood cells.

3. (Cancelled)

4. The method for sorting nucleated red blood cells according to claim 1 or 2,
wherein the search range is a range in which the white blood cell having a distance from the candidate cell is shortest and the white blood cell having a distance from the candidate cell becomes long are sequentially selected, and a number of the white blood cells which have been selected is 2 to 20.

5. (Amended) The method for sorting nucleated red blood cells according to any one of claims 1, 2 and 4,
wherein the spectral characteristics are absorbances in at least one monochromatic light beam selected from wavelengths included in a light wavelength region of 400 to 650 nm.

6. (Amended) The method for sorting nucleated red blood cells according to any one of claims 1, 2, 4 and 5,
wherein the candidate cell selection step includes a first step of selecting a cell of which a ratio of an area of a cell nucleus of the cell to an area of cytoplasm of the cell satisfies Formula 1, in a case where the area of cytoplasm of the cell is set to C and the area of the cell nucleus of the cell is set to N.

$$0.25 < N / C < 1.0 \qquad \text{Formula 1}$$

7. (Amended) The method for sorting nucleated red blood cells according to any one of claims 1, 2, 4, 5 and 6,
wherein the candidate cell selection step includes a second step of selecting a cell of which a ratio of an area of a cell nucleus to a square of a length of a major axis of a circular shape or a major axis of an elliptical shape satisfies Formula 2, in a case where the area of the cell nucleus of the cell is set to N and the length of the diameter of the circular shape circumscribing the cell nucleus of the cell or the major axis of the elliptical shape is set to L.

$$0.65 < N / (L \times L) < 0.785 \qquad \text{Formula 2}$$

## FIG. 1

```
┌─────────────────────────────┐
│      COLLECTION STEP         │ ─ S12
└─────────────────────────────┘
              │
              ▼
┌─────────────────────────────┐
│    CONCENTRATION STEP        │ ─ S14
└─────────────────────────────┘
              │
              ▼
┌─────────────────────────────┐
│      DEFINITION STEP         │ ─ S16
└─────────────────────────────┘
              │
              ▼
┌─────────────────────────────┐
│     AMPLIFICATION STEP       │ ─ S18
└─────────────────────────────┘
              │
              ▼
┌─────────────────────────────┐
│        SORTING STEP          │ ─ S20
└─────────────────────────────┘
              │
              ▼
┌─────────────────────────────┐
│     DETERMINATION STEP       │ ─ S22
└─────────────────────────────┘
```

## FIG. 2

```
┌─────────────────────────────┐
│      CANDIDATE CELL          │ ─ S32
│     SELECTION STEP           │
└─────────────────────────────┘
              │
              ▼
┌─────────────────────────────┐
│     WHITE BLOOD CELL         │ ─ S34
│     SELECTION STEP           │
└─────────────────────────────┘
              │
              ▼
┌─────────────────────────────┐
│  NUCLEATED RED BLOOD CELL    │ ─ S36
│     DEFINITION STEP          │
└─────────────────────────────┘
```

# FIG. 3

```
                                    S42
                 ┌──────────────────────┐
                 │   IS SUBJECT CELL?    │  No
                 │  (CLOSED BY LINE?)    │──────────┐
                 └──────────────────────┘          │
                         │ Yes                      │
                                    S44             │
                 ┌──────────────────────┐          │
                 │   IS THERE NUCLEUS?   │  No       │
                 │  (IS THERE DOT-SHAPED │──────────┤
                 │      SUBSTANCE?)      │          │
                 └──────────────────────┘          │
                         │ Yes                      │
                                    S46             │
                 ┌──────────────────────┐          │
                 │  IS SIZE OF NUCLEUS OF│  No       │
                 │  CELL WITHIN PRESCRIBED│─────────┤
                 │        RANGE?         │          │
                 └──────────────────────┘          │
                         │ Yes                      │
                                    S48             │
                 ┌──────────────────────┐          │
                 │   IS SHAPE OF NUCLEUS │  No       │
                 │  WITHIN PRESCRIBED    │──────────┤
                 │        RANGE?         │          │
                 └──────────────────────┘          │
                         │ Yes                      │
                 ┌───────────────┐        ┌──────────────────┐
                 │    SELECT     │        │  DO NOT SELECT   │
                 └───────────────┘        └──────────────────┘
```

## FIG. 4A

## FIG. 4B

## FIG. 5

## FIG. 6

| | |
|---|---|
| DETERMINE CANDIDATE CELL<br>(CANDIDATE CELL SELECTION STEP) | ~S72 |
| SET NUMBER OF WHITE BLOOD CELLS | ~S74 |
| PERFORM SEARCHING UNTIL NUMBER OF<br>BLOOD CELLS BECOMES PRESCRIBED RANGE | ~S76 |

# FIG. 7

# FIG. 8

| DETERMINE CANDIDATE CELL<br>(CANDIDATE CELL SELECTION STEP) | S82 |
| SET SEARCH RANGE (AREA) | S84 |
| SEARCH WHITE BLOOD CELL<br>WITHIN SEARCH RANGE | S86 |

# FIG. 9

```
┌─────────────────────────────────────────┐
│   MEASURE ABSORBANCE OF CANDIDATE CELL,  │
│   WHITE BLOOD CELL, AND PERIPHERY OF     │ ∼S92
│   CANDIDATE CELL AND WHITE BLOOD CELL    │
└─────────────────────────────────────────┘
                    │
                    ▼
┌─────────────────────────────────────────┐
│   OBTAIN TRUE VALUES OF CANDIDATE CELL   │
│          AND WHITE BLOOD CELL            │ ∼S94
└─────────────────────────────────────────┘
                    │
                    ▼
┌─────────────────────────────────────────┐
│   PROPORTION OF ABSORBANCE OF CANDIDATE  │
│      CELL IS DIVIDED BY PROPORTION OF    │ ∼S96
│     ABSORBANCE OF WHITE BLOOD CELL       │
└─────────────────────────────────────────┘
                    │
                    ▼
┌─────────────────────────────────────────┐
│       DEFINE NUCLEATED RED BLOOD CELL    │ ∼S98
└─────────────────────────────────────────┘
```

## INTERNATIONAL SEARCH REPORT

| | International application No. |
|---|---|
| | PCT/JP2015/067712 |

| A. CLASSIFICATION OF SUBJECT MATTER |
|---|
| *G01N33/49*(2006.01)i, *C12Q1/02*(2006.01)i, *G01N21/27*(2006.01)i, *G01N33/48* (2006.01)i, *G01N33/483*(2006.01)i, *C12Q1/68*(2006.01)n |

According to International Patent Classification (IPC) or to both national classification and IPC

| B. FIELDS SEARCHED |
|---|
| Minimum documentation searched (classification system followed by classification symbols)<br>G01N33/49, C12Q1/02, G01N21/27, G01N33/48, G01N33/483, C12Q1/68 |

| Documentation searched other than minimum documentation to the extent that such documents are included in the fields searched |
|---|
| Jitsuyo Shinan Koho      1922–1996   Jitsuyo Shinan Toroku Koho   1996–2015<br>Kokai Jitsuyo Shinan Koho   1971–2015   Toroku Jitsuyo Shinan Koho   1994–2015 |

| Electronic data base consulted during the international search (name of data base and, where practicable, search terms used) |
|---|
| JSTPlus/JMEDPlus/JST7580(JDreamIII) |

### C. DOCUMENTS CONSIDERED TO BE RELEVANT

| Category* | Citation of document, with indication, where appropriate, of the relevant passages | Relevant to claim No. |
|---|---|---|
| Y<br>A | WO 2013/075100 A1  (CELLSCAPE CORP.),<br>23 May 2013 (23.05.2013),<br>paragraphs [000219] to [000254]<br>& JP 2014-533509 A      & US 2013/0130265 A1<br>& EP 2780465 A1 | 1-2,5<br>3-4,6-7 |
| Y<br>A | WO 2014/030380 A1  (Fuji Xerox Co., Ltd.),<br>27 February 2014 (27.02.2014),<br>paragraphs [0035] to [0046]<br>& JP 2014-39504 A        & CN 104487564 A | 1-2,5<br>3-4,6-7 |
| A | JP 2005-506525 A  (Beckman Coulter, Inc.),<br>03 March 2005 (03.03.2005),<br>entire text; all drawings<br>& US 6410330 B1        & WO 2003/012429 A1<br>& EP 1412740 A1        & CN 1549925 A | 1-7 |

| ☐ Further documents are listed in the continuation of Box C. | ☐ See patent family annex. |
|---|---|

| * | Special categories of cited documents: |
|---|---|
| "A" | document defining the general state of the art which is not considered   to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search<br>    21 July 2015 (21.07.15) | Date of mailing of the international search report<br>    04 August 2015 (04.08.15) |
|---|---|
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012023298 A
- JP 2002514304 A
- JP 58115346 A
- JP S58115346 A
- JP 2014014485 A
- JP 2004248619 A
- JP 2004361158 A
- WO 2012166425 A2

**Non-patent literature cited in the description**

- **Y. L. ZHENG et al.** Prenatal diagnosis from maternal blood: simultaneous immunophenotyping and FISH of fetal nucleated erythrocytes isolated by negative magnetic cell sorting. *J Med. Genet.,* December 1993, vol. 30 (12), 1051-1056
- **Y. L. ZHENG et al.** Flow sorting of fetal erythroblasts using intracytoplasmic anti-fetal haemoglobin: preliminary observations on maternal samples. *Prenat Diagn,* October 1995, vol. 15 (10), 897-905